(19) Europäisches Patentamt
European Patent Office
Office européen des brevets

(11) Publication number: 0 027 482
A2

# (12) EUROPEAN PATENT APPLICATION

(21) Application number: 79302770.7

(22) Date of filing: 03.12.79

(51) Int. Cl.$^3$: A 01 N 47/42
C 07 D 401/12
//C07D213/71

(30) Priority: 22.10.79 US 83752

(43) Date of publication of application:
29.04.81 Bulletin 81/17

(84) Designated Contracting States:
BE DE FR GB IT LU NL

(71) Applicant: E.I. DU PONT DE NEMOURS AND COMPANY
Legal Department 1007 Market Street
Wilmington, Delaware 19898(US)

(72) Inventor: Yeh, Chin-Lung
116 Weldin Park Drive
Wilmington, Delaware(US)

(74) Representative: Hildyard, Edward Martin et al,
Frank B. Dehn & Co. Imperial House 15-19 Kingsway
London WC2B 6UZ(GB)

## (54) Herbicidal pyridinesulfonylisothioureas, preparation and use thereof, compositions containing them, intermediates for preparing them and preparation of such intermediates.

(57) Compounds of the general formula

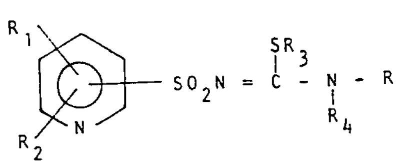
(1)

and their agriculturally suitable salts wherein:-

$R_1$ and $R_2$ are H or various substituents;

$R_3$ is $C_1$ - $C_5$ alkyl;

$R_4$ is H or $CH_3$; and

R is an optionally substituted pyrimidine or triazine residue, are of interest as pre-emergence and post-emergence herbicides and for the regulation of plant growth. They can be formulated for use in conventional manner. The compounds can be prepared by reacting novel intermediates of general formula

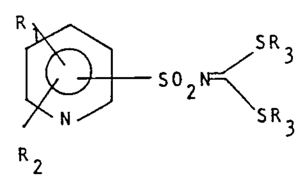
(11)

with an amino- or methylamino-substituted pyrimidine or triazine derivative. The intermediates can be prepared from the corresponding pyridinesulfonamides by reaction with carbon disulfide and an alkyl halide.

EP 0 027 482 A2

# Herbicidal pyridinesulfonylisothioureas, preparation and use thereof, compositions containing them, intermediates for preparing them and preparation of such intermediates

## Background of the Invention

This invention relates to pyridinesulfonylisothioureas which are useful as agricultural chemicals and to intermediates for making them.

French Patent No. 1,468,747 discloses the following *para*-substituted phenylsulfonamides, useful as antidiabetic agents:

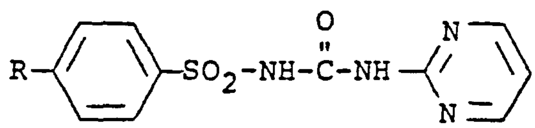

wherein R = H, halogen, $CF_3$ or alkyl.

Logemann et al. Chem. Ab., 53, 18052 g (1959), disclose a number of sulfonamides, including uracil derivatives and those having the formula:

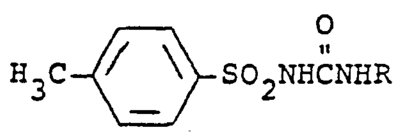

wherein R is butyl, phenyl or 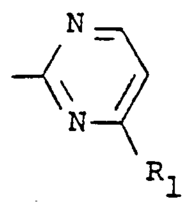

and $R_1$ is hydrogen or methyl. When tested for hypoglycemic effect in rats (oral doses of 25 mg/100 g), the compounds in which R is butyl and phenyl were most potent.

Wojciechowski, J. Acta. Polon. Pharm. 19, p. 125-5 (1962) [Chem. Ab., 59 1633 e] describes the synthesis of N-[(2,6-dimethoxypyrimidin-4-yl)-aminocarbonyl]-4-methylbenzenesulfonamide:

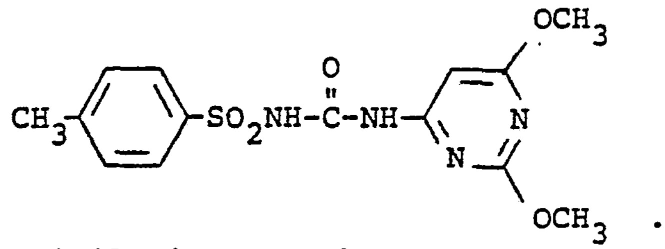

Based upon similarity to a known compound, the author predicted hypoglycemic activity for the foregoing compound.

Netherlands Patent 121,788, published September 15, 1966, teaches the preparation of compounds of Formula (i), and their use as general or selective herbicides,

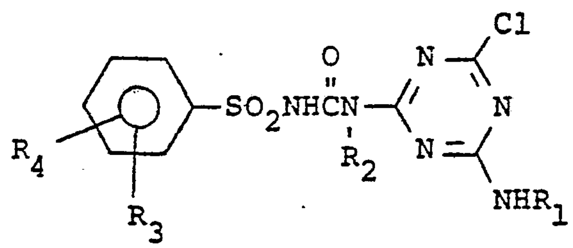
(i)

wherein

$R_1$ and $R_2$ may independently be alkyl of 1-4 carbon atoms; and

$R_3$ and $R_4$ may independently be hydrogen, chlorine or alkyl of 1-4 carbon atoms.

Compounds of Formula (ii), and their use as antidiabetic agents, are reported in J. Drug. Res. 6, 123 (1974).

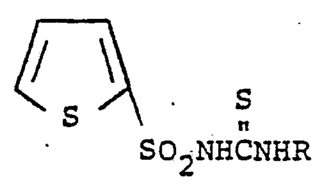
(ii)

wherein R is pyridyl.

United States Patent 3,689,549 F (September 5, 1972) to R. P. Williams discloses "heterocyclic sulfonamides wherein the heteroatoms are inert can be used, e.g. compounds having the furan, thiophene or pyridine nucleus," in the production of sulfonyl isocyanates from sulfonamides in a sulfolane solvent.

B. G. Boggiano, V. Petrow, O. Stephenson and A. M. Wild, in Journal of Pharmacy and Pharmacology 13, 567-574 (1961) disclose the following compounds which were tested for hypoglycemic activity.

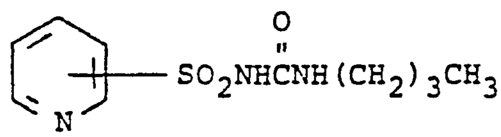

where $-SO_2NH\overset{O}{\overset{\|}{C}}NH(CH_2)_3CH_3$ is in the 2- or 3-position.

J. Delarge in Acta Pol. Pharm. 34, 245-249 (1977) discloses N-alkylcarbamoylpyridinesulfonamides, as described in the structure below, as mild anti-inflammatory agents and strong diuretics.

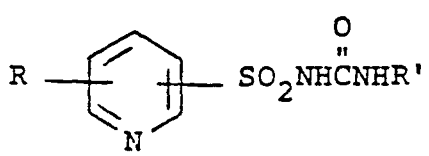

R = 3-, 4-, 5-, 6-Me, 2-, 4-, 6-Cl, 3-Br, 4-$ET_2N$, 4-$Me_2CHNH$, 4-$(3\text{-}ClC_6H_4)NH$, 4-$(3\text{-}CF_3C_6H_4)NH$

R' = Et, Pr, $Me_2CH$, Bu,

$SO_2NH\overset{O}{\overset{\|}{C}}NHR$ in the 2-, 3- and 4-position.

German Patent 2,516,025 (November 6, 1975) to J. E. Delarge, C. L. Lapiere and A. H. Georges discloses the following compounds as inflammation inhibitors and diuretics.

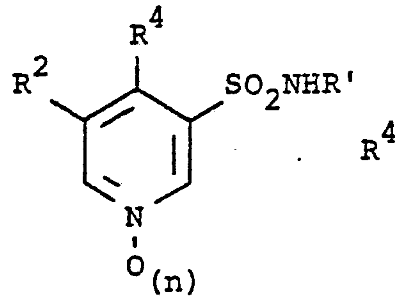

$R^4 = XR$

$R = C_6H_4R^3$ ($R^3$ = Cl, $CF_3$, Me, MeO, H, Br, F, $NO_2$, Et, $NH_2$), Et, iso-Pr, 4-methylfuryl, $C_6H_3Cl_2$-, $C_6H_3(CF_3)Cl$;

R' = alkylcarbamoyl, cyclohexylcarbamoyl, arylcarbamoyl, $CSNHCH_2CH{=}CH_2$, $CONHC_6H_4Cl$-p, alkylthiocarbamoyl, H, COEt;

$R^2$ = H, Me;

X = NH, NMe, O, S, NEt; and

n = 0, 1.

United States Patent 3,346,590 (October 10, 1967) (to K. Dickere and E. Kühle) discloses the following pyridinesulfonyl isothiocyanates as novel compounds.

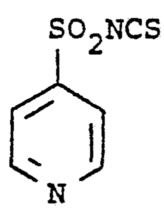

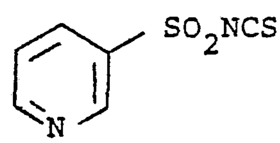

The presence of undesired vegetation causes substantial damage to useful crops, especially agricultural products that satisfy man's basic food and fiber needs, such as cotton, rice, corn, wheat, soybean and the like. The current population explosion and concomitant world food and fiber shortage demand improvements in the efficiency of producing these crops.

Preventing or minimizing the loss of a portion of such valuable crops by killing or inhibiting the growth of undesired vegetation is one way of improving this efficiency.

A wide variety of materials useful for killing or inhibiting (controlling) the growth of undesired vegetation is available; such materials are commonly referred to as herbicides. The need exists, however, for still more effective herbicides that destroy or control weeds without causing significant damage to useful crops.

## Summary of the Invention

According to this invention, there are provided novel compounds of Formula I , suitable agricultural compositions containing them and methods of using them as pre-emergence and post-emergence herbicides and as plant growth regulants. There are also provided novel intermediates of Formula II which are useful in preparing the novel herbicidal compounds of Formula I; the compounds of Formula I are also useful as intermediates for the preparation of the herbicidal ureas disclosed in our copending European application corresponding to U.S. Serial Nos. 966,258 and 083,753

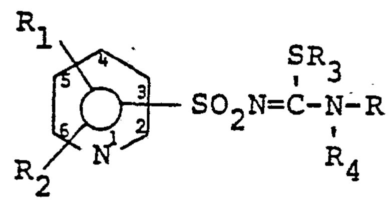

I

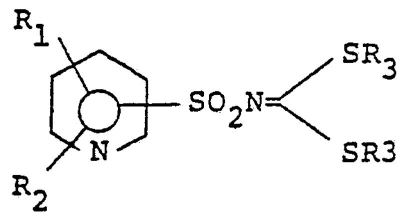

II

wherein

R is 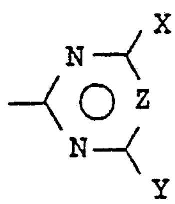 , 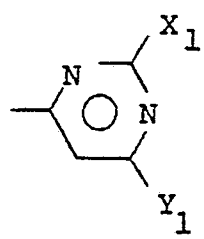 or 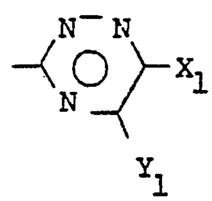 ;

$R_1$ is H, Cl, Br, F, $C_1$-$C_4$ alkyl, $C_1$-$C_4$ alkoxy, $C_1$-$C_4$ alkylthio, $NO_2$ or $\overset{O}{\overset{\|}{C}}OR_5$;

$R_2$ is H, Cl, Br or $CH_3$;

$R_3$ is $C_1$-$C_5$ alkyl;

$R_4$ is H or $CH_3$;

$R_5$ is $C_1$-$C_6$ alkyl, $C_3$-$C_6$ alkenyl, $CH_2CH_2OCH_3$, $CH_2CH_2OCH_2CH_3$, $CH_2CH_2CH_2OCH_3$ or $CH_2CH_2Cl$;

X is $CH_3$, $CH_3O$- or $CH_3CH_2O$-;

Y is H, Cl, $CH_3$, $CF_3$, $NHCH_3$, $-N(CH_3)_2$, $-CH_2OR_{10}$, $CH_2CH_2OR_{10}$, $-OCH_2CF_3$ or $VR_9$;

Z is CH or N;

V is oxygen or sulfur;

$R_9$ is $CH_3$, $-CH_2CH_3$, $-CH_2CH_2OR_{10}$, $-CH_2CO_2R_{10}$, $-\underset{CH_3}{CH}CO_2R_{10}$ or $-CH_2CH_2CO_2R_{10}$;

$R_{10}$ is $CH_3$ or $CH_3CH_2$

$Y_1$ is H, $OCH_3$ or $CH_3$; and

$X_1$ is H, Cl, $-OCH_3$, $OCH_2CH_3$ or $CH_3$;

and their agriculturally suitable salts; providing that (1) $X_1$ and $Y_1$ are not simultaneously H and (2) when R =

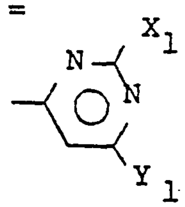

or

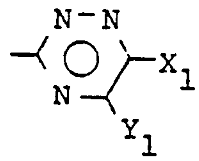

then $R_4$ is H.

Preferred in order of increasing activity and/or increasingly favorable cost are:

(1) Compounds of the generic scope wherein $R_4$ is hydrogen;

(2) Compounds of preferred (1) wherein $R_2$ is hydrogen; and

(3) Compounds of preferred (2) wherein $R_1$ is chlorine.

Equally preferred in order of increasing activity and/or increasingly favorable cost are:

(4) Compounds of the generic scope wherein R is

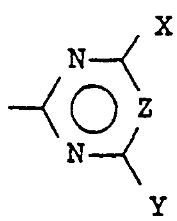

;

(5) Compounds of preferred (4) wherein X is $-CH_3$ or $-OCH_3$ and Y is $CH_3$, $-OCH_3$ or $-OCH_2CH_3$.

More preferred for their higher activity and/or more favorable cost are those compounds of preferred (5) wherein $R_4$ is hydrogen, e.g. the compound of Example 2.

Compounds of Formula II can be prepared as shown in Equation 1.

Equation 1

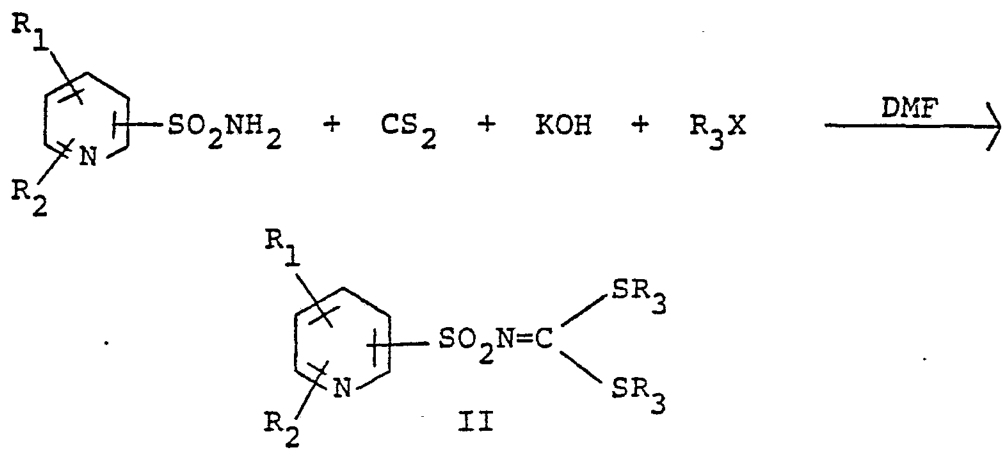


II

N-(Pyridinylsulfonyl)carbonimidodithioic acids, dialkyl esters of Formula II, can be conveniently prepared by the reaction of an appropriate pyridinesulfonamide, carbon disulfide, an alkali metal hydroxide and an alkyl halide in a suitable solvent, such as dimethylformamide or dimethylsulfoxide at ambient temperature. Good yields can be obtained if alkali metal hydroxide and carbon disulfide are added in portions to the solution of pyridinesulfonamide. The reaction is generally exothermic and the desired product can be isolated by pouring the reaction mixture into cold water and filtering off the precipitated solid. Generally, the reaction temperature is between 15 and 40°C, preferably 0 and 100°C. Ambient pressure is preferred.

Compounds of Formula I can be prepared as shown in Equation 2.

Equation 2

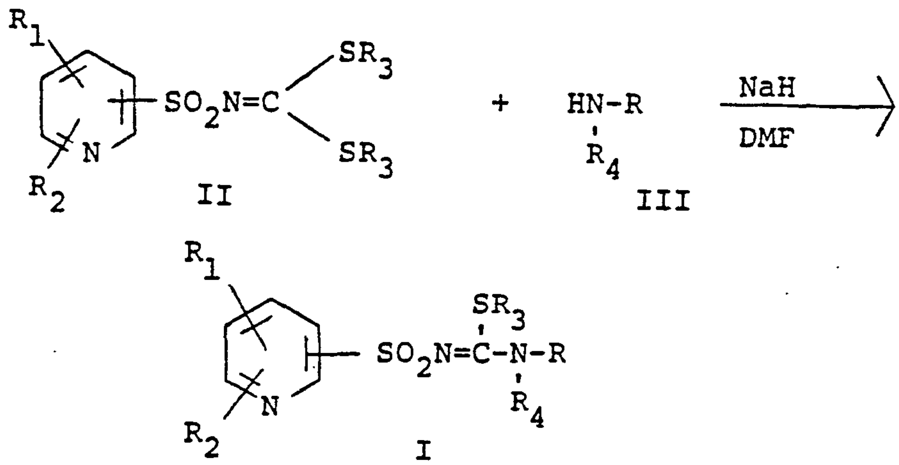


II III

I

This reaction is best carried out in inert aprotic solvents, such as tetrahydrofuran, N,N-dimethylformamide, or dimethylacetamide at ambient temperature. The anion of aminopyrimidine or aminotriazine can be prepared by the reaction of an alkali metal hydride and an appropriate amine III. It is often convenient to add the compounds of Formula II to a stirred solution of the anion species at ambient temperature. Addition of water and adjustment of the pH to above 3 with HCl, followed by filtration yields the compounds of Formula I. Compounds of Formula I may be converted into their acid addition salts by reaction with agriculturally suitable acids.

The following example further illustrates the preparation of intermediates of Formula II; these compounds likewise can form acid addition salts.

Example 1

N-(3-Pyridinylsulfonyl)carbonimidodithioic acid, dimethyl ester

To a stirred solution of 15.8 g of pyridinesulfonamide in 70 ml of N,N-dimethylformamide was added 6.6 g of powdered potassium hydroxide. The mixture was stirred for 50 minutes at room temperature and 3 ml of carbon disulfide was added dropwise. The solution was then stirred at room temperature for one hour, with two portions of 3.3 g of powdered potassium hydroxide and 1.5 ml of carbondisulfide added at half hour intervals. After an additional 0.5 hour of stirring, the mixture was cooled to 0-5°C, and 13 ml of iodomethane was added dropwise. The resulting mixture was stirred overnight, poured onto 250 g of ice-water and the precipitate was filtered off. It was washed with water until neutral and then dried at reduced pressure to give 17 g of the desired product, m.p. 115-118°C.

By using a procedure of Example 1 with the appropriate sulfonamide, the following compounds of Table I can be prepared.

Table I

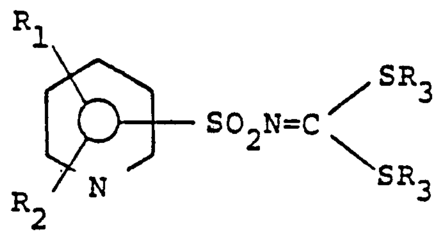

| $R_1$ | $R_2$ | $R_3$ | Position 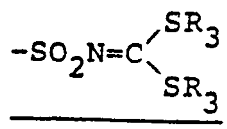 |
|---|---|---|---|
| 5-Cl | H | $CH_3$ | 2 |
| 6-Cl | H | $CH_3$ | 2 |
| 6-Cl | 3-$CH_3$ | $CH_3$ | 2 |
| 6-Br | 3-$CH_3$ | $CH_3$ | 2 |
| 5-Br | H | $CH_3$ | 2 |
| 5-Br | 3-Br | $CH_3$ | 2 |
| 3-Br | H | $CH_3$ | 2 |
| 4-$OC_2H_5$ | 3-Br | $CH_3$ | 2 |
| 3-F | H | $CH_3$ | 2 |
| 4-$OCH_3$ | H | $CH_3$ | 2 |
| 5-F | H | $CH_3$ | 2 |
| 3-$CO_2CH_3$ | H | $CH_2CH_3$ | 2 |
| 4-$CO_2$-n-$C_6H_{13}$ | H | $CH_3$ | 2 |
| 3-$OCH_3$ | Cl | $CH_3$ | 2 |
| 6-F | H | $CH_3$ | 2 |
| 5-$NO_2$ | H | $CH_3$ | 2 |
| 3-$NO_2$ | H | $CH_3$ | 2 |
| 4-$CH_3$ | H | $CH_3$ | 2 |
| 4-$C_2H_5$ | H | $CH_3$ | 2 |
| 3-$CH_3$ | H | $CH_2CH_2CH_2CH_3$ | 2 |
| 6-Cl | H | $CH_3$ | 3 |
| 2-$CH_3$ | 6-$CH_3$ | $CH_3$ | 3 |
| 5-$CH_3$ | H | $CH_3$ | 3 |
| 6-$CH_3$ | H | $CH_3$ | 3 |

Table I (continued)

| $R_1$ | $R_2$ | $R_3$ | Position $-SO_2N=C(SR_3)_2$ |
|---|---|---|---|
| $5-C_2H_5$ | $2-CH_3$ | $CH_3$ | 3 |
| 5-$\underline{n}$-$C_4H_9$ | $2-CH_3$ | $CH_3$ | 3 |
| 2-Cl | 6-Cl | $CH_3$ | 3 |
| $4-CO_2C_2H_5$ | H | $CH_3$ | 3 |
| $5-CO_2CH_3$ | H | $CH_3$ | 3 |
| $6-CO_2$-$\underline{i}$-$C_3H_7$ | H | $CH_3$ | 3 |
| $4-OCH_3$ | H | $CH_3$ | 3 |
| 6-$\underline{n}$-$C_4H_9$ | H | $CH_3$ | 3 |
| $5-CH_3$ | 6-Cl | $CH_2CH_2CH_3$ | 3 |
| $5-NO_2$ | 6-Cl | $CH_2CH_3$ | 3 |
| 4-Cl | H | $CH_3$ | 3 |
| 6-F | H | $CH_3$ | 3 |
| 6-Br | H | $CH_3$ | 3 |
| $6-SC_2H_5$ | H | $CH_3$ | 3 |
| $4-SC_4H_9$ | H | $CH_3$ | 3 |
| $2-SC_2H_5$ | H | $CH_3$ | 3 |
| 4-F | H | $CH_3$ | 3 |
| 4-Br | H | $CH_3$ | 3 |
| $6-SCH_3$ | H | $CH_3$ | 3 |
| $5-NO_2$ | 6-Cl | $CH_3$ | 3 |
| 5-Br | H | $CH(CH_3)_2$ | 3 |
| $5-NO_2$ | H | $CH_3$ | 3 |
| 4-$\underline{n}$-$C_4H_9$ | H | $CH_3$ | 3 |
| 6-$\underline{n}$-$C_4H_9$ | H | $CH_3$ | 3 |
| 5-F | 6-Cl | $CH_3$ | 3 |
| $5-NO_2$ | 6-Br | $CH_3$ | 3 |
| $2-C_2H_5$ | H | $CH_3$ | 4 |
| $2-CH_3$ | $6-CH_3$ | $CH_3$ | 4 |
| 3-Cl | 5-Cl | $CH_3$ | 4 |
| 3-Br | 5-Br | $CH_3$ | 4 |
| H | 3-Cl | $CH_3$ | 4 |

Table I (continued)

| $R_1$ | $R_2$ | $R_3$ | Position $-SO_2N=C(SR_3)_2$ |
|---|---|---|---|
| 3-Br | H | $CH_3$ | 4 |
| $3-CH_3$ | H | $CH_3$ | 4 |
| $2-CO_2-\underline{n}-C_6H_{13}$ | H | $CH_3$ | 4 |
| $5-NO_2$ | 2-Cl | $CH_3$ | 4 |
| H | H | $CH_3$ | 2 |
| 3-Cl | H | $CH_3$ | 2 |
| $3-CO_2CH_3$ | H | $CH_3$ | 2 |
| $3-CO_2-\underline{i}-C_3H_7$ | H | $CH_3$ | 2 |
| $3-CH_3$ | H | $CH_3$ | 2 |
| $3-NO_2$ | H | $CH_3$ | 2 |
| 3-Cl | H | $CH_3$ | 2 |
| $3-CO_2CH_3$ | H | $CH_3$ | 2 |
| $3-CO_2-\underline{i}-C_3H_7$ | H | $CH_3$ | 2 |
| $3-CH_3$ | H | $CH_2CH_2CH_3$ | 2 |
| $3-NO_2$ | H | $CH_2CH_2CH_3$ | 2 |
| H | H | $CH_2CH_2CH_3$ | 3 |
| H | H | $CH_2CH_2CH_3$ | 3 |
| $2-NO_2$ | H | $CH_2CH_2CH_3$ | 3 |
| $2-CO_2-\underline{i}-C_3H_7$ | H | $CH_2CH_2CH_3$ | 3 |
| $2-CO_2CH_3$ | H | $CH_2CH_2CH_3$ | 3 |
| H | H | $CH_2CH_2CH_3$ | 3 |
| 2-Cl | H | $CH_2CH_2CH_3$ | 3 |
| $2-NO_2$ | H | $CH_2CH_2CH_3$ | 3 |
| $2-CO_2-\underline{i}-C_3H_7$ | H | $CH_2CH_2CH_3$ | 3 |
| $2-CO_2CH_3$ | H | $CH_2CH_2CH_3$ | 3 |
| $2-CH_3$ | H | $CH_2CH_2CH_3$ | 3 |
| $2-CH_3$ | H | $CH_2CH_2CH_3$ | 3 |
| H | H | $CH_2CH_3$ | 3 |
| H | H | $CH_2CH_2CH_2CH_3$ | 3 |

Table I (continued)

| $R_1$ | $R_2$ | $R_3$ | Position $-SO_2N=C\begin{smallmatrix}SR_3\\SR_3\end{smallmatrix}$ |
|---|---|---|---|
| 4-Cl | H | $CH_3$ | 3 |
| $4-NO_2$ | H | $CH_3$ | 3 |
| $4-NO_2$ | H | $CH_3$ | 3 |
| $4-CH_3$ | H | $CH_3$ | 3 |
| $4-CH_3$ | H | $CH_3$ | 3 |
| $4-CO_2CH_3$ | H | $CH_2CH_2CH_2CH_3$ | 3 |
| $4-CO_2CH_3$ | H | $CH_3$ | 3 |
| $4-CO_2-\underline{i}-C_3H_7$ | H | $CH_3$ | 3 |
| $4-CO_2-\underline{i}-C_3H_7$ | H | $CH_3$ | 3 |
| H | H | $CH_3$ | 4 |
| H | H | $CH_3$ | 4 |
| 3-Cl | H | $CH_3$ | 4 |
| 3-Cl | H | $CH_3$ | 4 |
| $3-NO_2$ | H | $CH_3$ | 4 |
| $3-NO_2$ | H | $CH_3$ | 4 |
| $3-CH_3$ | H | $CH_3$ | 4 |
| $3-CH_3$ | H | $CH_3$ | 4 |
| $3-CO_2CH_3$ | H | $CH_3$ | 4 |
| $3-CO_2CH_3$ | H | $CH_3$ | 4 |
| $3-CO_2-\underline{i}-C_3H_7$ | H | $CH_3$ | 4 |
| $3-CO_2-\underline{i}-C_3H_7$ | H | $CH_2CH_2CH_3$ | 4 |

The following example illustrates the preparation of compounds of Formula I.

## Example 2

**N-(4-Methoxy-6-methyl-1,3,5-triazin-2-yl)-N'-(3-pyridinylsulfonyl)carbamimidothioic acid, methyl ester**

To a suspension of 0.6 g of sodium hydride (50% oil dispersion) in 25 ml of dry N,N-dimethylformamide was added 1.75 g of 2-amino-4-methoxy-6-methyl-1,3,5-triazine. The resulting yellow suspension was stirred at room temperature for 3 hours and then added to 3.95 g of 3-pyridinylsulfonylcarbonimidodithionic acid dimethyl ester. The reddish slurry was stirred at room temperature for 2 hours, poured into 250 g of ice-water and filtered to remove 0.6 g of the unreacted starting material. Hydrochloric acid (10%) was added to adjust the pH to 4 and the precipitated product was filtered off. After washing with water until neutral, the desired product was dried at reduced pressure to give 1.5 g of the isothiourea, m.p. 128-130°C.

By using the procedure of Example 2 with an equivalent amount of an aminopyrimidine, or aminotriazine and appropriately substituted compounds of Formula II, the compounds of Tables II, III, IIIa and IIIb can be prepared.

Table II

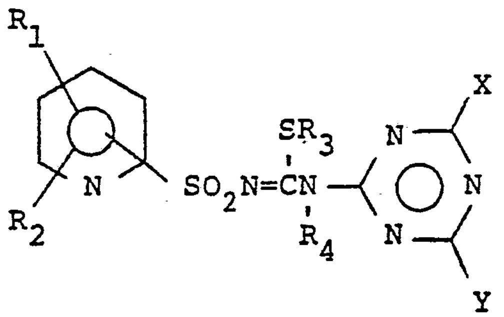

| $R_1$ | $R_2$ | $R_3$ | $R_4$ | Position $-SO_2N$ | X | Y |
|---|---|---|---|---|---|---|
| 5-Cl | H | $CH_3$ | H | 2 | $CH_3$ | H |
| 6-Cl | H | $CH_3$ | H | 2 | H | Cl |
| 6-Cl | 3-$CH_3$ | $CH_3$ | H | 2 | $OCH_3$ | $OCH_3$ |
| 6-Br | 3-$CH_3$ | $CH_3$ | H | 2 | $CH_3$ | $OCH_3$ |
| 5-Br | H | $CH_3$ | H | 2 | $CH_3$ | $OCH_3$ |
| 5-Br | 3-Br | $CH_3$ | H | 2 | $OCH_3$ | $OCH_3$ |
| 5-F | H | $CH_3$ | $CH_3$ | 2 | $CH_3$ | $CH_2OCH_3$ |
| 3-Br | H | $CH_3$ | $CH_3$ | 2 | $OCH_3$ | $OCH_3$ |
| 4-$OC_2H_5$ | 3-Br | $CH_2CH_3$ | H | 2 | $CH_3$ | $OC_2H_5$ |
| 3-F | H | $CH_3$ | H | 2 | $CH_3$ | $OCH_3$ |
| 3-$OCH_3$ | H | $CH_3$ | $CH_3$ | 2 | $CH_3$ | $CH_2OCH_3$ |
| 6-F | H | $CH_3$ | H | 2 | $CH_3$ | $OCH_2CF_3$ |
| 5-$NO_2$ | H | $CH_3$ | H | 2 | $CH_3$ | $N(CH_3)_2$ |
| 4-$CH_3$ | H | $CH_3$ | H | 2 | $CH_3$ | Cl |
| 4-$C_2H_5$ | H | $CH_3$ | H | 2 | $CH_3$ | $CF_3$ |
| 2-Cl | H | $CH_2CH_2CH_3$ | $CH_3$ | 3 | $CH_3$ | $OCH_3$ |
| 6-Cl | H | $CH_3$ | H | 3 | $CH_3$ | $OCH_3$ |
| 2-$CH_3$ | 6-$CH_3$ | $CH_3$ | H | 3 | $CH_3$ | $OCH_3$ |
| 4-$CH_3$ | H | $CH_3$ | $CH_3$ | 3 | $CH_3$ | $OCH_2CH_2OCH_3$ |
| 2-$CO_2CH_2CH_2Cl$ | H | $CH_3$ | H | 3 | $CH_3$ | $OCH_3$ |
| 2-$CO_2CH_2CH{=}CH_2$ | H | $CH_3$ | H | 3 | $CH_3$ | $OCH_3$ |
| 2-$CO_2CH_2CH_2OCH_3$ | H | $CH_3$ | H | 3 | $OCH_3$ | $OCH_3$ |

Table II (continued)

| $R_1$ | $R_2$ | $R_3$ | $R_4$ | Position $-SO_2N$ | X | Y |
|---|---|---|---|---|---|---|
| 5-$CH_3$ | H | $CH_3$ | $CH_3$ | 3 | $CH_3$ | $OCH_2CH_2CH_2OCH_3$ |
| 6-$CH_3$ | H | $CH_3$ | H | 3 | $CH_3$ | $OCH_2CO_2CH_3$ |
| 5-$C_2H_5$ | 2-$CH_3$ | $CH_3$ | H | 3 | $CH_3$ | $OCH_2CO_2C_2H_5$ |
| 5-Br | 6-Cl | $CH_3$ | H | 3 | $CH_3$ | $SCH_2CO_2CH_3$ |
| 2-$CO_2CH_3$ | H | $CH_3$ | H | 3 | $CH_3$ | $S(CH_2)_2CO_2C_2H_5$ |
| 4-$CO_2C_2H_5$ | H | $CH_3$ | H | 3 | $OCH_3$ | $OCH_3$ |
| 6-$CO_2CH_3$ | H | $CH_2(CH_3)_2$ | H | 3 | $CH_3$ | $OCH_3$ |
| 4-$OCH_3$ | H | $CH_3$ | H | 3 | $CH_3$ | $OCH_3$ |
| 6-$OCH_3$ | H | $CH_3$ | H | 3 | $OC_2H_5$ | $OCH_3$ |
| 5-$CH_3$ | 6-Cl | $CH_3$ | H | 3 | $CH_3$ | $CF_3$ |
| 5-$NO_2$ | 6-Cl | $CH_3$ | H | 3 | $CH_3$ | $OCH_2CF_3$ |
| 4-Cl | H | $CH_3$ | H | 3 | $CH_3$ | $N(CH_3)_2$ |
| 6-Br | H | $CH_3$ | H | 3 | $CH_3$ | Cl |
| 6-$OC_2H_5$ | H | $CH_3$ | H | 3 | $CH_3$ | $SC_2H_5$ |
| 4-S-n-$C_4H_9$ | H | $CH_3$ | H | 3 | $OCH_3$ | $OCH_3$ |
| 2-$SC_2H_5$ | H | $CH_3$ | H | 3 | $CH_3$ | $OCH_3$ |
| 4-E | H | $CH_3$ | H | 3 | $CH_3$ | $OCH_2CH_2CO_2C_2H_5$ |
| 6-$SCH_3$ | H | $CH_3$ | H | 3 | $CH_3$ | $OCH_3$ |
| 5-$NO_2$ | 6-Cl | $CH_3$ | $CH_3$ | 3 | $OCH_3$ | $OCH_3$ |
| 5-Br | H | $CH_2CH_2CH_2CH_3$ | H | 3 | $OCH_3$ | $OCH_3$ |
| 5-$NO_2$ | H | $CH_3$ | H | 3 | $CH_3$ | $CF_3$ |
| 6-n-$C_4H_9$ | H | $CH_3$ | H | 3 | $OCH_3$ | $OCH_3$ |
| 5-F | 6-Cl | $CH_3$ | H | 3 | $OC_2H_5$ | $CH_2OCH_3$ |
| 2-$C_2H_5$ | H | $CH_3$ | $CH_3$ | 4 | $CH_3$ | $OCH_3$ |
| 2-$C_2H_5$ | H | $CH_3$ | H | 4 | $CH_3$ | $OC_2H_5$ |
| 2-$CH_3$ | 6-$CH_3$ | $CH_3$ | H | 4 | $CH_3$ | $OCH_2CH_2OCH_3$ |
| 3-Cl | 5-Cl | $CH_3$ | H | 4 | $CH_3$ | $OCH_2CH_2CO_2CH_3$ |
| 3-Cl | 5-Cl | $CH_3$ | $CH_3$ | 4 | $OCH_3$ | $OCH_3$ |
| 3-Cl | H | $CH_3$ | H | 4 | $CH_3$ | Cl |
| 3-Br | H | $CH_3$ | H | 4 | $OCH_3$ | $OCH_3$ |
| 3-$CH_3$ | H | $CH_3$ | H | 4 | $CH_3$ | $SC_2H_5$ |
| 2-$CO_2$-n-$C_4H_9$ | H | $CH_3$ | H | 4 | $CH_3$ | $OCH(CH_3)CO_2CH_3$ |

Table II (continued)

| $R_1$ | $R_2$ | $R_3$ | $R_4$ | Position $-SO_2N$ | X | Y |
|---|---|---|---|---|---|---|
| 5-$NO_2$ | 2-Cl | $CH_3$ | H | 4 | $CH_3$ | $OCH_3$ |
| 5-Cl | H | $CH_3$ | H | 4 | $CH_3$ | $OCH_3$ |
| H | H | $CH_2CH_3$ | H | 2 | $CH_3$ | $CH_3$ |
| H | H | $CH_3$ | H | 2 | $CH_3$ | $OCH_3$ |
| H | H | $CH_3$ | H | 2 | $OCH_3$ | $OCH_3$ |
| 3-Cl | H | $CH_3$ | H | 2 | $CH_3$ | $OCH_3$ |
| 3-Cl | H | $CH_3$ | H | 2 | $CH_3$ | $CH_3$ |
| 3-$NO_2$ | H | $CH_3$ | H | 2 | $CH_3$ | $CH_3$ |
| 3-$NO_2$ | H | $CH_3$ | H | 2 | $CH_3$ | $OCH_3$ |
| 3-$CO_2CH_3$ | H | $CH_3$ | H | 2 | $OCH_3$ | $OCH_3$ |
| 3-$CO_2CH_3$ | H | $CH_2CH_2CH_3$ | H | 2 | $CH_3$ | $OCH_3$ |
| 3-$CO_2CH_3$ | H | $CH_3$ | H | 2 | $CH_3$ | $CH_3$ |
| 3-$CO_2$-i-$C_4H_9$ | H | $CH_3$ | H | 2 | $CH_3$ | $CH_3$ |
| 3-$CH_3$ | H | $CH_3$ | H | 2 | $OCH_3$ | $OCH_3$ |
| 3-$CH_3$ | H | $CH_3$ | H | 2 | $CH_3$ | $OCH_3$ |
| 3-$CH_3$ | H | $CH_3$ | H | 2 | $CH_3$ | $CH_3$ |
| H | H | $CH_3$ | H | 3 | $CH_3$ | $CH_3$ |
| H | H | $CH_3$ | H | 3 | $OCH_3$ | $OCH_3$ |
| 2-Cl | H | $CH_3$ | H | 3 | $OCH_3$ | $OCH_3$ |
| 2-Cl | H | $CH_3$ | H | 3 | $CH_3$ | $CH_2OCH_3$ |
| 2-Cl | H | $CH_3$ | H | 3 | $CH_3$ | $CH_3$ |
| 2-$NO_2$ | H | $CH_3$ | H | 3 | $CH_3$ | $CH_3$ |
| 2-$NO_2$ | H | $CH_3$ | H | 3 | $CH_3$ | $OCH_3$ |
| 2-$NO_2$ | H | $CH_3$ | H | 3 | $OCH_3$ | $OCH_3$ |
| 2-$CO_2CH_3$ | H | $CH_3$ | H | 3 | $OCH_3$ | $OCH_3$ |
| 2-$CO_2CH_3$ | H | $CH_3$ | H | 3 | $CH_3$ | $OCH_3$ |
| 2-$CO_2CH_3$ | H | $CH_3$ | H | 3 | $CH_3$ | $CH_3$ |
| 2-$CO_2$-i-$C_3H_7$ | H | $CH_3$ | H | 3 | $CH_3$ | $CH_3$ |
| 2-$CO_2$-i-$C_3H_7$ | H | $CH_3$ | H | 3 | $CH_3$ | $OCH_3$ |
| 2-$CO_2$-i-$C_3H_7$ | H | $CH_3$ | H | 3 | $OCH_3$ | $OCH_3$ |

Table II (continued)

| $R_1$ | $R_2$ | $R_3$ | $R_4$ | Position $-SO_2N$ | X | Y |
|---|---|---|---|---|---|---|
| 2-$CH_3$ | H | $CH_3$ | H | 3 | $OCH_3$ | $OCH_3$ |
| 2-$CH_3$ | H | $CH_3$ | H | 3 | $CH_3$ | $OCH_3$ |
| 2-$CH_3$ | H | $CH_3$ | H | 3 | $CH_3$ | $CH_3$ |
| 4-Cl | H | $CH_3$ | H | 3 | $CH_3$ | $CH_3$ |
| 4-Cl | H | $CH_3$ | H | 3 | $OCH_3$ | $OCH_3$ |
| 4-$NO_2$ | H | $CH_3$ | H | 3 | $OCH_3$ | $OCH_3$ |
| 4-$NO_2$ | H | $CH_3$ | H | 3 | $CH_3$ | $OCH_3$ |
| 4-$NO_2$ | H | $CH_3$ | H | 3 | $CH_3$ | $CH_3$ |
| 4-$CO_2CH_3$ | H | $CH_3$ | H | 3 | $CH_3$ | $CH_3$ |
| 4-$CO_2CH_3$ | H | $CH_3$ | H | 3 | $CH_3$ | $OCH_3$ |
| 4-$CO_2CH_3$ | H | $CH_3$ | H | 3 | $OCH_3$ | $OCH_3$ |
| 4-$CO_2$-i-$C_3H_7$ | H | $CH_3$ | H | 3 | $CH_3$ | $CH_3$ |
| 4-$CO_2$-i-$C_3H_7$ | H | $CH_3$ | H | 3 | $OCH_3$ | $OCH_3$ |
| 4-$CO_2$-i-$C_3H_7$ | H | $CH_3$ | H | 3 | $CH_3$ | $OCH_3$ |
| 4-$CH_3$ | H | $CH_3$ | H | 3 | $CH_3$ | $CH_3$ |
| 4-$CH_3$ | H | $CH_2CH_2CH_3$ | H | 3 | $OCH_3$ | $OCH_3$ |
| H | H | $CH_3$ | H | 4 | $OCH_3$ | $OCH_3$ |
| H | H | $CH_3$ | H | 4 | $CH_3$ | $OCH_3$ |
| H | H | $CH_3$ | H | 4 | $CH_3$ | $CH_3$ |
| 3-Cl | H | $CH_3$ | H | 4 | $CH_3$ | $CH_3$ |
| 3-$NO_2$ | H | $CH_3$ | H | 4 | $OCH_3$ | $OCH_3$ |
| 3-$NO_2$ | H | $CH_3$ | H | 4 | $CH_3$ | $OCH_3$ |
| 3-$CO_2CH_3$ | H | $CH_3$ | H | 4 | $OCH_3$ | $OCH_3$ |
| 3-$CO_2CH_3$ | H | $CH_3$ | H | 4 | $CH_3$ | $CH_3$ |
| 3-$CO_2$-i-$C_3H_7$ | H | $CH_3$ | H | 4 | $CH_3$ | $CH_3$ |
| 3-$CO_2$-i-$C_3H_7$ | H | $CH_3$ | H | 4 | $CH_3$ | $OCH_3$ |
| 3-$CO_2$-i-$C_3H_7$ | H | $CH_2CH_2CH_2CH_2CH_3$ | H | 4 | $OCH_3$ | $OCH_3$ |
| 3-$CH_3$ | H | $CH_3$ | H | 4 | $CH_3$ | $CH_3$ |
| 3-$CH_3$ | H | $CH_3$ | H | 4 | $CH_3$ | $OCH_3$ |
| 3-$CH_3$ | H | $CH_3$ | H | 4 | $OCH_3$ | $OCH_3$ |

Table III

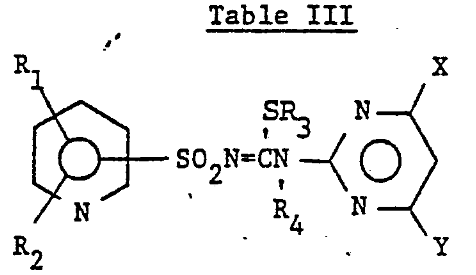

| $R_1$ | $R_2$ | $R_3$ | $R_4$ | Position $-SO_2N$ | X | Y |
|---|---|---|---|---|---|---|
| 5-Cl | H | $CH_3$ | H | 2 | $CH_3$ | H |
| 6-Cl | H | $CH_3$ | H | 2 | H | Cl |
| 6-Cl | $3-CH_3$ | $CH_3$ | H | 2 | $OCH_3$ | $OCH_3$ |
| 6-Br | $3-CH_3$ | $CH_3$ | H | 2 | $CH_3$ | $OCH_3$ |
| 5-Br | H | $CH_3$ | H | 2 | $CH_3$ | $OCH_3$ |
| 5-Br | 3-Br | $CH_3$ | H | 2 | $OCH_3$ | $OCH_3$ |
| 3-Br | H | $CH_3$ | $CH_3$ | 2 | $OCH_3$ | $OCH_3$ |
| $4-OC_2H_5$ | 3-Br | $CH_3$ | H | 2 | $CH_3$ | $OC_2H_5$ |
| 3-F | H | $CH_3$ | H | 2 | $CH_3$ | $OCH_3$ |
| 5-F | H | $CH_3$ | $CH_3$ | 2 | $CH_3$ | $CH_2OCH_3$ |
| $3-OCH_3$ | H | $CH_3$ | $CH_3$ | 2 | $CH_3$ | $CH_2OCH_3$ |
| 6-F | H | $CH_2CH_3$ | H | 2 | $CH_3$ | $OCH_2CF_3$ |
| $5-NO_2$ | H | $CH_3$ | H | 2 | $CH_3$ | $N(CH_3)_2$ |
| $4-CH_3$ | H | $CH_3$ | H | 2 | $CH_3$ | Cl |
| $4-C_2H_5$ | H | $CH_3$ | H | 2 | $CH_3$ | $CF_3$ |
| 2-Cl | H | $CH_3$ | $CH_3$ | 3 | $CH_3$ | $OCH_3$ |
| 6-Cl | H | $CH_3$ | H | 3 | $CH_3$ | $OCH_3$ |
| $2-CH_3$ | $6-CH_3$ | $CH_3$ | H | 3 | $CH_3$ | $OCH_3$ |
| $4-CH_3$ | H | $CH_3$ | $CH_3$ | 3 | $CH_3$ | $OCH_2CH_2OCH_3$ |
| 6-Cl | H | $CH_3$ | H | 3 | $CH_3$ | $CH_3$ |
| 6-Cl | H | $CH_3$ | H | 3 | $OCH_3$ | $OCH_3$ |
| $2-CO_2CH_2CH=CH_2$ | H | $CH_3$ | H | 3 | $CH_3$ | $OCH_3$ |
| $2-CO_2CH-CH=CH(CH_2)_2CH_3$ | H | $CH_2CH_2CH_3$ | H | 3 | $CH_3$ | $OCH_3$ |
| $2-CO_2CH_2CH_2OCH_3$ | H | $CH_3$ | H | 3 | $CH_3$ | $CH_3$ |
| $2-CO_2CH_2CH_2OCH_2CH_3$ | H | $CH_3$ | H | 3 | $CH_3$ | $OCH_3$ |
| $2-CO_2CH_2CH_2CH_2OCH_3$ | H | $CH_3$ | H | 3 | $CH_3$ | $OCH_3$ |
| $2-CO_2CH_2CH_2Cl$ | H | $CH_3$ | H | 3 | $OCH_3$ | $OCH_3$ |

Table III (continued)

| $R_1$ | $R_2$ | $R_3$ | $R_4$ | Position $-SO_2N$ | X | Y |
|---|---|---|---|---|---|---|
| 5-$CH_3$ | H | $CH_3$ | $CH_3$ | 3 | $CH_3$ | $OCH_2CH_2CH_2OCH_3$ |
| 6-$CH_3$ | H | $CH_3$ | H | 3 | $CH_3$ | $OCH_2CO_2CH_3$ |
| 5-$C_2H_5$ | 2-$CH_3$ | $CH_3$ | H | 3 | $CH_3$ | $OCH_2CO_2C_2H_5$ |
| 5-Br | 6-Cl | $CH_3$ | H | 3 | $CH_3$ | $SCH_2CO_2CH_3$ |
| 2-$CO_2CH_3$ | H | $CH_3$ | H | 3 | $CH_3$ | $S(CH_2)_2CO_2C_2H_5$ |
| 4-$CO_2C_2H_5$ | H | $CH_3$ | H | 3 | $OCH_3$ | $OCH_3$ |
| 6-$CO_2CH_3$ | H | $CH_3$ | H | 3 | $CH_3$ | $OCH_3$ |
| 4-$OCH_3$ | H | $CH_3$ | H | 3 | $CH_3$ | $OCH_3$ |
| 6-$OCH_3$ | H | $CH_3$ | H | 3 | $OC_2H_5$ | $OCH_3$ |
| 5-$CH_3$ | 6-Cl | $CH_3$ | H | 3 | $CH_3$ | $CF_3$ |
| 5-$NO_2$ | 6-Cl | $CH_3$ | H | 3 | $CH_3$ | $OCH_2CF_3$ |
| 4-Cl | H | $CH_3$ | H | 3 | $CH_3$ | $N(CH_3)_2$ |
| 6-Br | H | $CH_2CH_2CH_2CH_2CH_3$ | H | 3 | $CH_3$ | Cl |
| 6-$OC_2H_5$ | H | $CH_3$ | H | 3 | $CH_3$ | $SC_2H_5$ |
| 4-S-n-$C_4H_9$ | H | $CH_3$ | H | 3 | $OCH_3$ | $OCH_3$ |
| 2-$SC_2H_5$ | H | $CH_3$ | H | 3 | $CH_3$ | $OCH_3$ |
| 4-F | H | $CH_3$ | H | 3 | $CH_3$ | $OCH_2CH_2CO_2C_2H_5$ |
| 6-$SCH_3$ | H | $CH_3$ | H | 3 | $CH_3$ | $OCH_3$ |
| 5-$NO_2$ | 6-Cl | $CH_3$ | $CH_3$ | 3 | $OCH_3$ | $OCH_3$ |
| 5-Br | H | $CH_3$ | H | 3 | $OCH_3$ | $OCH_3$ |
| 5-$NO_2$ | H | $CH_3$ | H | 3 | $CH_3$ | $CF_3$ |
| 6-n-$C_4H_9$ | H | $CH_3$ | H | 3 | $OCH_3$ | $OCH_3$ |
| 5-F | 6-Cl | $CH_3$ | H | 3 | $OC_2H_5$ | $CH_2OCH_3$ |
| 2-Cl | 4-Cl | $CH_3$ | H | 3 | $OCH_3$ | $OCH_3$ |
| 2-Cl | 4-Cl | $CH_3$ | H | 3 | $CH_3$ | $OCH_3$ |
| 2-$C_2H_5$ | H | $CH_3$ | $CH_3$ | 4 | $CH_3$ | $OCH_3$ |
| 2-$C_2H_5$ | H | $CH_3$ | H | 4 | $CH_3$ | $OC_2H_5$ |
| 2-$CH_3$ | 6-$CH_3$ | $CH_3$ | H | 4 | $CH_3$ | $OCH_2CH_2OCH_3$ |
| 3-Cl | 5-Cl | $CH_3$ | H | 4 | $CH_3$ | $OCH_2CH_2CO_2CH_3$ |
| 3-Cl | 5-Cl | $CH_3$ | $CH_3$ | 4 | $OCH_3$ | $OCH_3$ |
| 3-Cl | H | $CH_3$ | H | 4 | $CH_3$ | Cl |

Table III (continued)

| $R_1$ | $R_2$ | $R_3$ | $R_4$ | Position $-SO_2N$ | X | Y |
|---|---|---|---|---|---|---|
| 3-Br | H | $CH_3$ | H | 4 | $OCH_3$ | $OCH_3$ |
| 3-$CH_3$ | H | $CH_3$ | H | 4 | $CH_3$ | $SC_2H_5$ |
| 2-$CO_2$-n-$C_4H_9$ | H | $CH_3$ | H | 4 | $CH_3$ | $OCH(CH_3)CO_2CH_3$ |
| 5-$NO_2$ | 2-Cl | $CH_3$ | H | 4 | $CH_3$ | $OCH_3$ |
| 5-Cl | H | $CH_3$ | H | 4 | $CH_3$ | $OCH_3$ |
| H | H | $CH(CH_3)_2$ | H | 2 | $CH_3$ | $CH_3$ |
| H | H | $CH_3$ | H | 2 | $CH_3$ | $OCH_3$ |
| H | H | $CH_3$ | H | 2 | $OCH_3$ | $OCH_3$ |
| 3-Cl | H | $CH_3$ | H | 2 | $CH_3$ | $OCH_3$ |
| 3-Cl | H | $CH_3$ | H | 2 | $CH_3$ | $CH_3$ |
| 3-$NO_2$ | H | $CH_3$ | H | 2 | $CH_3$ | $CH_3$ |
| 3-$NO_2$ | H | $CH_3$ | H | 2 | $CH_3$ | $OCH_3$ |
| 3-$CO_2CH_3$ | H | $CH_3$ | H | 2 | $OCH_3$ | $OCH_3$ |
| 3-$CO_2CH_3$ | H | $CH_3$ | H | 2 | $CH_3$ | $OCH_3$ |
| 3-$CO_2CH_3$ | H | $CH_3$ | H | 2 | $CH_3$ | $CH_3$ |
| 3-$CO_2$-i-$C_3H_7$ | H | $CH_3$ | H | 2 | $CH_3$ | $CH_3$ |
| 3-$CH_3$ | H | $CH_3$ | H | 2 | $OCH_3$ | $OCH_3$ |
| 3-$CH_3$ | H | $CH_3$ | H | 2 | $CH_3$ | $OCH_3$ |
| 3-$CH_3$ | H | $CH_3$ | H | 2 | $CH_3$ | $CH_3$ |
| H | H | $CH_3$ | H | 3 | $CH_3$ | $CH_3$ |
| H | H | $CH_3$ | H | 3 | $OCH_3$ | $OCH_3$ |
| 2-Cl | H | $CH_3$ | H | 3 | $OCH_3$ | $OCH_3$ |
| 2-Cl | H | $CH_3$ | H | 3 | $CH_3$ | $-CH_2OCH_3$ |
| 2-Cl | H | $CH_3$ | H | 3 | $CH_3$ | $CH_3$ |
| 2-$NO_2$ | H | $CH_3$ | H | 3 | $CH_3$ | $CH_3$ |
| 2-$NO_2$ | H | $CH_3$ | H | 3 | $CH_3$ | $OCH_3$ |
| 2-$NO_2$ | H | $CH_3$ | H | 3 | $OCH_3$ | $OCH_3$ |
| 2-$CO_2CH_3$ | H | $CH_3$ | H | 3 | $OCH_3$ | $OCH_3$ |
| 2-$CO_2CH_3$ | H | $CH_3$ | H | 3 | $CH_3$ | $OCH_3$ |
| 2-$CO_2CH_3$ | H | $CH_3$ | H | 3 | $CH_3$ | $CH_3$ |

Table III (continued)

| $R_1$ | $R_2$ | $R_3$ | $R_4$ | Position $-SO_2N$ | X | Y |
|---|---|---|---|---|---|---|
| $2-CO_2-\underline{i}-C_3H_7$ | H | $CH_3$ | H | 3 | $CH_3$ | $CH_3$ |
| $2-CO_2-\underline{i}-C_3H_7$ | H | $CH_3$ | H | 3 | $CH_3$ | $OCH_3$ |
| $2-CO_2-\underline{i}-C_3H_7$ | H | $CH_3$ | H | 3 | $OCH_3$ | $OCH_3$ |
| $2-CH_3$ | H | $CH_2CH_2CH_2CH_3$ | H | 3 | $OCH_3$ | $OCH_3$ |
| $2-CH_3$ | H | $CH_3$ | H | 3 | $CH_3$ | $OCH_3$ |
| $2-CH_3$ | H | $CH_3$ | H | 3 | $CH_3$ | $CH_3$ |
| 4-Cl | H | $CH_3$ | H | 3 | $CH_3$ | $CH_3$ |
| 4-Cl | H | $CH_3$ | H | 3 | $OCH_3$ | $OCH_3$ |
| $4-NO_2$ | H | $CH_3$ | H | 3 | $OCH_3$ | $OCH_3$ |
| $4-NO_2$ | H | $CH_3$ | H | 3 | $CH_3$ | $OCH_3$ |
| $4-NO_2$ | H | $CH_3$ | H | 3 | $CH_3$ | $CH_3$ |
| $4-CO_2CH_3$ | H | $CH_3$ | H | 3 | $CH_3$ | $CH_3$ |
| $4-CO_2CH_3$ | H | $CH_3$ | H | 3 | $CH_3$ | $OCH_3$ |
| $4-CO_2CH_3$ | H | $CH_3$ | H | 3 | $OCH_3$ | $OCH_3$ |
| $4-CO_2-\underline{i}-C_3H_7$ | H | $CH_2CH_3$ | H | 3 | $CH_3$ | $CH_3$ |
| $4-CO_2-\underline{i}-C_3H_7$ | H | $CH_3$ | H | 3 | $OCH_3$ | $OCH_3$ |
| $4-CO_2-\underline{i}-C_3H_7$ | H | $CH_3$ | H | 3 | $CH_3$ | $OCH_3$ |
| $4-CH_3$ | H | $CH_3$ | H | 3 | $CH_3$ | $CH_3$ |
| $4-CH_3$ | H | $CH_3$ | H | 3 | $OCH_3$ | $OCH_3$ |
| H | H | $CH_3$ | H | 4 | $OCH_3$ | $OCH_3$ |
| H | H | $CH_3$ | H | 4 | $CH_3$ | $OCH_3$ |
| H | H | $CH_3$ | H | 4 | $CH_3$ | $CH_3$ |
| 3-Cl | H | $CH_3$ | H | 4 | $CH_3$ | $CH_3$ |
| $3-NO_2$ | H | $CH_3$ | H | 4 | $OCH_3$ | $OCH_3$ |
| $3-NO_2$ | H | $CH_3$ | H | 4 | $CH_3$ | $OCH_3$ |
| $3-CO_2CH_3$ | H | $CH_3$ | H | 4 | $OCH_3$ | $OCH_3$ |
| $3-CO_2CH_3$ | H | $CH_2CH_3$ | H | 4 | $CH_3$ | $CH_3$ |
| $3-CO_2-\underline{i}-C_3H_7$ | H | $CH_3$ | H | 4 | $CH_3$ | $CH_3$ |
| $3-CO_2-\underline{i}-C_3H_7$ | H | $CH_3$ | H | 4 | $CH_3$ | $OCH_3$ |
| $3-CO_2-\underline{i}-C_3H_7$ | H | $CH_3$ | H | 4 | $OCH_3$ | $OCH_3$ |
| $3-CH_3$ | H | $CH_3$ | H | 4 | $CH_3$ | $CH_3$ |
| $3-CH_3$ | H | $CH_3$ | H | 4 | $CH_3$ | $OCH_3$ |
| $3-CH_3$ | H | $CH_3$ | H | 4 | $OCH_3$ | $OCH_3$ |

Table IIIa

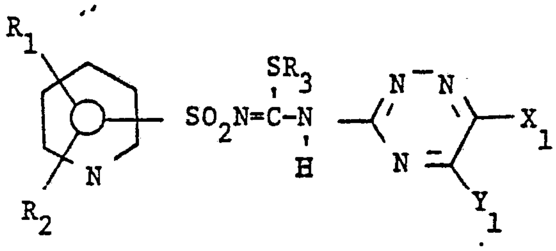

| $R_1$ | $R_2$ | $R_3$ | Position $-SO_2N=$ | $Y_1$ | $X_1$ |
|---|---|---|---|---|---|
| 5-Cl | H | $CH_3$ | 2 | $CH_3$ | $CH_3$ |
| 6-Cl | 3-$CH_3$ | $CH_3$ | 2 | $CH_3$ | $CH_3$ |
| 5-Br | 3-Br | $CH_3$ | 2 | $OCH_3$ | $OCH_3$ |
| 4-$OC_2H_5$ | 3-Br | $CH_3$ | 2 | $OCH_3$ | $OCH_3$ |
| 5-F | H | $CH_3$ | 2 | $CH_3$ | $CH_3$ |
| 4-$C_2H_5$ | H | $CH_3$ | 2 | $CH_3$ | $OCH_3$ |
| 2-Cl | H | $CH_3$ | 3 | $CH_3$ | $OCH_3$ |
| 5-$C_2H_5$ | 2-$CH_3$ | $CH_3$ | 3 | $CH_3$ | $CH_3$ |
| 5-Br | 6-Cl | $CH_3$ | 3 | $CH_3$ | $OCH_3$ |
| 4-$CO_2C_2H_5$ | H | $CH_3$ | 3 | $CH_3$ | $CH_3$ |
| 6-$CO_2CH_3$ | H | $CH_3$ | 3 | $CH_3$ | $CH_3$ |
| 6-$OCH_3$ | H | $CH_3$ | 3 | $CH_3$ | $OCH_3$ |
| 6-Br | H | $CH_3$ | 3 | $OCH_3$ | $OCH_3$ |
| 5-$NO_2$ | 6-Cl | $CH_3$ | 3 | $CH_3$ | $CH_3$ |
| 2-$C_2H_5$ | H | $CH_2CH_2CH_3$ | 4 | $CH_3$ | $CH_3$ |
| 3-Cl | 5-Cl | $CH_3$ | 4 | $CH_3$ | $CH_3$ |
| 3-Br | H | $CH_3$ | 4 | $OCH_3$ | $OCH_3$ |

Table IIIb

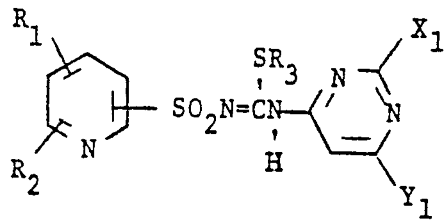

| $R_1$ | $R_2$ | $R_3$ | Position of $-SO_2N=$ | $Y_1$ | $X_1$ |
|---|---|---|---|---|---|
| 3-Br | H | $CH_3$ | 4 | $OCH_3$ | $CH_3$ |
| 3-Cl | 5-Cl | $CH_3$ | 4 | $CH_3$ | $OCH_3$ |
| $2-C_2H_5$ | H | $CH_3$ | 4 | $OCH_3$ | $OCH_3$ |
| 6-F | $5-NO_2$ | $CH_3$ | 3 | $OCH_3$ | $OCH_3$ |
| 6-$\underline{n}$-$C_4H_9$ | H | $CH_3$ | 3 | $OCH_3$ | $CH_3$ |
| $5-NO_2$ | H | $CH_3$ | 3 | $OCH_3$ | $OCH_3$ |
| $5-NO_2$ | 6-Cl | $CH_3$ | 3 | $OCH_3$ | $OCH_3$ |
| 4-F | H | $CH_3$ | 3 | $OCH_3$ | $OC_2H_5$ |
| $6-SC_2H_5$ | H | $CH_2CH_2CH_2CH_2CH_3$ | 3 | $OCH_3$ | $OCH_3$ |
| $6-CO_2CH_3$ | H | $CH_3$ | 3 | $OCH_3$ | $OCH_2CH_3$ |
| $4-CO_2CH_3$ | H | $CH_3$ | 3 | $OCH_3$ | Cl |
| 2-Cl | H | $CH_3$ | 3 | $OCH_3$ | $OCH_3$ |
| $4-C_2H_5$ | H | $CH_2CH_3$ | 2 | $OCH_3$ | $OCH_3$ |
| $5-NO_2$ | H | $CH_3$ | 2 | $OCH_3$ | $OCH_3$ |
| $4-CO_2CH_3$ | H | $CH_3$ | 2 | $OCH_3$ | $OCH_3$ |
| 5-Cl | H | $CH_3$ | 2 | $OCH_3$ | $OCH_3$ |

Formulations

Useful formulations of the compounds of Formula I can be prepared in conventional ways. They include dusts, granules, pellets, suspensions, emulsions, wettable powders, emulsifiable concentrates and the like. Many of them can be applied directly. Sprayable formulations can be extended in suitable media and used at spray volumes of from a few liters to several hundred liters per hectare. High strength compositions are used primarily as concentrates which are to be diluted prior to ultimate use. The formulations, broadly, contain about 0.1% to 99% by weight of active ingredient(s) and at least one of a) about 0.1% to 20% surfactant(s) and b) about 1% to 99.9% solid or liquid diluent(s). More specifically, they will contain these ingredients in the approximate proportions set forth in Table IV.

Table IV

| | Weight Percent* | | |
|---|---|---|---|
| | Ingredient | Diluent(s) | Surfactant(s) |
| Wettable Powders | 20-90 | 0-74 | 1-10 |
| Oil Suspensions, Solutions, Emulsions (including Emulsifiable Concentrates) | 5-50 | 40-95 | 0-15 |
| Aqueous Suspensions | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

*Active Ingredient plus at least one of a Surfactant or a Diluent equals 100 weight percent.

Lower or higher levels of active ingredient can be present, depending on the intended use and the physical properties of the compound. Higher ratios of

surfactant to active ingredient are sometimes desirable, and are achieved by incorporation into the formulation, or by tank mixing.

Some typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending, and usually grinding, as in a hammer or fluid energy mill. Suspensions are prepared by wet milling (see, for example, Littler, U.S. Patent 3,060,084). Granules and pellets can be made by spraying the active material on preformed granular carriers or by agglomeration techniques. See J. E. Browning, "Agglomeration", Chemical Engineering, Dec. 4, 1967, pp. 147ff. and "Perry's Chemical Engineer's Handbook", 4th Ed., McGraw-Hill, New York, 1963, pp. 8-59ff.

For further information regarding the art of formulation, see for example:

H. M. Loux, U.S. Patent 3,235,361, Col. 6, line 16 through Col. 7, line 19 and Examples 10 through 41.

R. W. Luckenbaugh, U.S. Patent 3,309,192, Col. 5, line 43 through Col. 7, line 62 and Examples 8, 12, 15, 39, 41, 52, 53, 58, 132, 138-140, 162-164, 166, 167 and 169-182.

H. Gysin and E. Knusli, U.S. Patent 2,891,855, Col. 3, line 66 through Col. 5, line 17 and Examples 1-4.

G. C. Klingman, "Weed Control as a Science", John Wiley & Sons, Inc., New York, 1961, pp. 81-96.

J. D. Fryer and S. A. Evans, "Weed Control Handbook", 5th Ed., Blackwell Scientific Publications, Oxford, 1968, pp. 101-103.

Unless indicated otherwise, all parts are by weight in the following examples.

Example 3

Wettable Powder

| | |
|---|---|
| N-(4-Methoxy-6-methyl-1,3,5-triazin-2-yl)-N'-(3-pyridinylsulfonyl)carbamimidothioic acid, methyl ester | 50% |
| sodium alkylnaphthalene-sulfonate | 2% |
| low viscosity methyl cellulose | 2% |
| diatomaceous earth | 46% |

The ingredients are blended, coarsely hammer-milled and then air milled to produce particles of active essentially all below 10 microns in diameter. The product is reblended before packaging.

Example 4

Granule

| | |
|---|---|
| wettable powder of Example 3 | 10% |
| attapulgite granules (U.S.S. #20-40; 0.84-0.42 mm) | 90% |

A slurry of wettable powder containing 50% solids is sprayed on the surface of attapulgite granules in a double-cone blender. The granules are dried and packaged.

Example 5

Wettable Powder

| | |
|---|---|
| N-(4-Methoxy-6-methyl-1,3,5-triazin-2-yl)-N'-(3-pyridinylsulfonyl)carbamimidothioic acid, methyl ester | 80% |
| sodium alkylnaphthalenesulfonate | 2% |
| sodium ligninsulfonate | 2% |
| synthetic amorphous silica | 3% |
| kaolinite | 13% |

The ingredients are thoroughly blended and hammer milled to produce particles essentially all below 50 microns. The product is reblended and sifted through a U.S.S. No. 50 sieve (0.3 mm opening) before packaging.

Example 6

Oil Suspensions

| | |
|---|---|
| N-(4-Methoxy-6-methyl-1,3,5-triazin-2-yl)-N'-(3-pyridinylsulfonyl)carbamimidothioic acid, methyl ester | 35% |
| blend of polyalcohol carboxylic esters and oil soluble petroleum sulfonates | 6% |
| xylene | 59% |

The ingredients are combined and ground together in a sand mill to produce particles essentially all below 3 microns. The product can be used directly, extended with oils, or emulsified in water.

Example 7

Extruded Pellet

| | |
|---|---|
| N-(4-Methoxy-6-methyl-1,3,5-triazin-2-yl)-N'-(3-pyridinylsulfonyl)carbamimidothioic acid, methyl ester | 25% |
| anhydrous sodium sulfate | 10% |
| crude calcium ligninsulfonate | 5% |
| sodium alkylnaphthalene-sulfonate | 1% |
| calcium/magnesium bentonite | 59% |

The ingredients are blended, hammer milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 8

Aqueous Suspension

| | |
|---|---|
| N-(4-Methoxy-6-methyl-1,3,5-triazin-2-yl)-N'-(3-pyridinylsulfonyl)carbamimidothioic acid, methyl ester | 50.0% |
| polyacrylic acid thickener | 0.3% |
| dodecylphenol polyethylene glycol ether | 0.5% |
| disodium phosphate | 1.0% |
| monosodium phosphate | 0.5% |
| polyvinyl alcohol | 1.0% |
| water | 46.7% |

The ingredients are ground together in a sand mill to produce particles essentially all under five microns in size.

Example 9

| | |
|---|---|
| N-(4-Methoxy-6-methyl-1,3,5-triazin-2-yl)-N'-(3-pyridinylsulfonyl)carbamimidothioic acid, methyl ester | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged,

screened to the desired size range, generally 14-100 mesh (1410-149 microns), and packaged for use.

Example 10

High Strength Concentrate

| | |
|---|---|
| N-(4-Methoxy-6-methyl-1,3,5-triazin-2-yl)-N'-(3-pyridinylsulfonyl)carbamimidothioic acid, methyl ester | 99% |
| trimethylnonyl polyethylene glycol ether | 1% |

The surfactant is sprayed upon the active ingredient in a blender and the mixture sifted through a U.S.S. No. 40 sieve (0.42 mm openings) prior to packaging. The concentrate may be formulated further for practical use.

Example 11

Wettable Powder

| | |
|---|---|
| N-(4-Methoxy-6-methyl-1,3,5-triazin-2-yl)-N'-(3-pyridinylsulfonyl)carbamimidothioic acid, methyl ester | 40% |
| dioctyl sodium sulfosuccinate | 1.5% |
| sodium ligninsulfonate | 3% |
| low viscosity methyl cellulose | 1.5% |
| attapulgite | 54% |

The ingredients are thoroughly blended, passed through an air mill, to produce an average particle size under 15 microns, reblended, and sifted through a U.S.S. No. 50 sieve (0.3 mm opening) before packaging.

All compounds of the invention may be formulated in the same manner.

## Example 12

Wettable Powder

| | |
|---|---|
| N-(4-Methoxy-6-methyl-1,3,5-triazin-2-yl)-N'-(3-pyridinylsulfonyl)carbamimidothioic acid, methyl ester | 65% |
| dodecylphenol polyethylene glycol ether | 2% |
| sodium ligninsulfonate | 4% |
| sodium silicoaluminate | 6% |
| montmorillonite (calcined) | 23% |

The ingredients are thoroughly blended. The liquid surfactant is added by spraying upon the solid ingredients in the blender. After grinding in a hammer mill to produce particles essentially all below 100 microns, the material is reblended and sifted through a U.S.S. No. 50 sieve (0.3 mm opening) and packaged.

## Example 13

Oil Suspension

| | |
|---|---|
| N-(4-Methoxy-6-methyl-1,3,5-triazin-2-yl)-N'-(3-pyridinylsulfonyl)carbamimidothioic acid, methyl ester | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Utility

The compounds of the present invention possess herbicidal activity and are useful for preparing compounds with herbicidal activity. The herbicides have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures.

The rates of application for the compounds of the invention are determined by a number of factors, including the types of weeds to be controlled, weather and climate, formulations selected, mode of application, amount of foliage present, etc. In general terms, the subject compounds should be applied at levels of around 0.5 to 10 kg/ha, the lower rates being suggested for use on lighter soils and/or those having a low organic matter content or for situations where only short-term persistence is required.

The compounds of the invention may be used in combination with any other commercial herbicide examples of which are those of the triazine, triazole, uracil, urea, amide, diphenylether, carbamate and bipyridylium types.

The herbicidal properties of the subject compounds were discovered in a number of greenhouse tests. The test procedures and results follow.

Test A

Seeds of crabgrass (*Digitaria* spp.), barnyardgrass (*Echinochloa crusgalli*), wild oats (*Avena fatua*), *Cassia tora*, morningglory (*Ipomoea* spp.), cocklebur (*Xanthium* spp.), sorghum, corn, soybean, rice, wheat as well as nutsedge tubers were planted in a growth medium and treated pre-emergence with the chemicals dissolved in a non-phytotoxic solvent. At the same time, cotton having five leaves (including cotyledonary ones), bush beans with the third trifoliate leaf expanding, crabgrass, barnyardgrass and wild oats with two leaves, cassia with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three-five leaves were sprayed. Treated plants and controls were maintained in a greenhouse for sixteen days, whereupon all species were compared to controls and visually rated for response to treatment. The ratings are based on a numerical scale extending from 0 = no injury, to 10 = complete kill. The accompanying descriptive symbols have the following meanings:

G = growth retardation;
C = chlorosis/necrosis;
6Y = abscised buds or flowers;
S = albinism; and
H = formative effects.

The ratings for the compounds tested by this procedure are presented in Table A. It will be seen that certain of the compounds tested have utility for selective post-emergence weed control in wheat.

Table A

| | 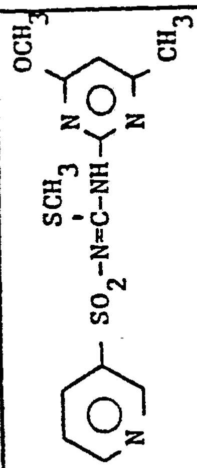 | 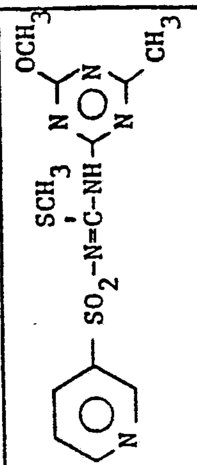 | 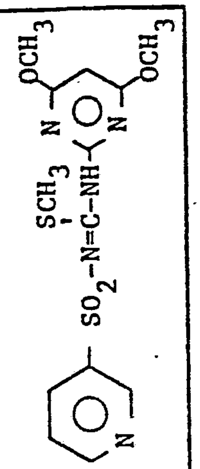 |
|---|---|---|---|
| kg/ha | 2 | 2 | 2 |
| POST-EMERGENCE | | | |
| BUSHBEAN | 3C,3G | 5S,8G,6Y | 1C,5G |
| COTTON | 4C,7G | 3C,3H | 3C,6G |
| MORNINGGLORY | 3C,8G | 1C | 1C |
| COCKLEBUR | 0 | 2C | 0 |
| CASSIA | 1C | 0 | 1C |
| NUTSEDGE | 5G | 0 | 2G |
| CRABGRASS | 2G | 4G | 0 |
| BARNYARDGRASS | 3G | 2C,6H | 0 |
| WILD OATS | 0 | 0 | 0 |
| WHEAT | 0 | 0 | 0 |
| CORN | 3G | 4H | 0 |
| SOYBEAN | 2C,4H | 2C | 1C |
| RICE | 1C,5G | 5G | 0 |
| SORGHUM | 2G | 1C,6G | 0 |
| PRE-EMERGENCE | | | |
| MORNINGGLORY | 7G | 1C,8G | 1C |
| COCKLEBUR | 7G | 3C | 0 |
| CASSIA | 1C,4G | 2C | 2C |
| NUTSEDGE | 7G | 1C | 5G |
| CRABGRASS | 5G | 1C,3G | 0 |
| BARNYARDGRASS | 1C,8H | 2C,8H | 2G |
| WILD OATS | 8G | 1C,4G | 0 |
| WHEAT | 1C,5G | 1C,8G | 0 |
| CORN | 2C,8G | 2C,7G | 2C,4G |
| SOYBEAN | 2C,3G | 1C | 0 |
| RICE | 9G | 1C,8G | 1C,5G |
| SORGHUM | 2H,8G | 2C,9G | 1C |

Table A (continued)

| | 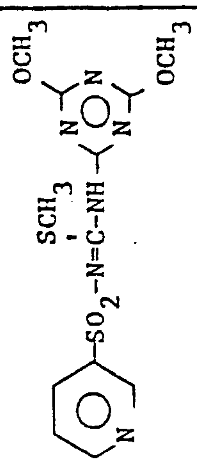 |
|---|---|
| kg/ha | 2 |
| POST-EMERGENCE | |
| BUSHBEAN | 1C |
| COTTON | 1C |
| MORNINGGLORY | 1C,5H |
| COCKLEBUR | 0 |
| CASSIA | 0 |
| NUTSEDGE | 0 |
| CRABGRASS | 3G |
| BARNYARDGRASS | 2C,6G |
| WILD OATS | 0 |
| WHEAT | 0 |
| CORN | 0 |
| SOYBEAN | 1C,2H |
| RICE | 3G |
| SORGHUM | 8G |
| PRE-EMERGENCE | |
| MORNINGGLORY | 8H |
| COCKLEBUR | 8G |
| CASSIA | 4G |
| NUTSEDGE | 0 |
| CRABGRASS | 3G |
| BARNYARDGRASS | 7H |
| WILD OATS | 0 |
| WHEAT | 8G |
| CORN | 1C,7G |
| SOYBEAN | 2G |
| RICE | 7H |
| SORGHUM | 8H |

Claims:

1. A compound of the formula:

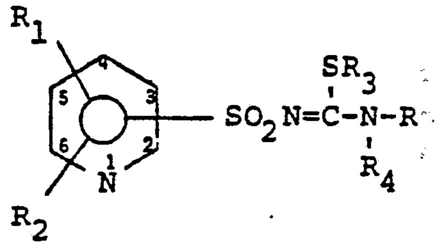

(I)

or

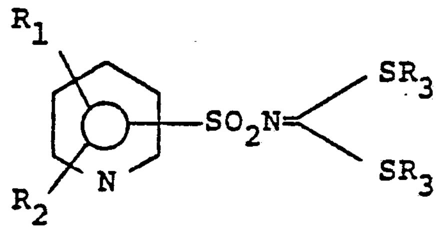

(II)

wherein

R is 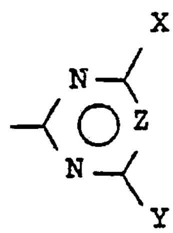 , 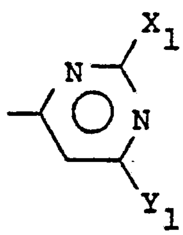 or 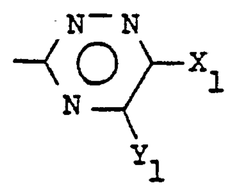 ;

$R_1$ is H, Cl, Br, F, $C_1$-$C_4$ alkyl, $C_1$-$C_4$ alkoxy, $C_1$-$C_4$ alkylthio, $NO_2$ or $\overset{O}{\overset{\|}{C}}OR_5$;

$R_2$ is H, Cl, Br or $CH_3$;

$R_3$ is $C_1$-$C_5$ alkyl;

$R_4$ is H or $CH_3$;

$R_5$ is $C_1$-$C_6$ alkyl, $C_3$-$C_6$ alkenyl, $CH_2CH_2OCH_3$, $CH_2CH_2OCH_2CH_3$, $CH_2CH_2CH_2OCH_3$ or $CH_2CH_2Cl$;

X is $CH_3$, $CH_3O$- or $CH_3CH_2O$-;

Y is H, Cl, $CH_3$, $CF_3$, $NHCH_3$, $-N(CH_3)_2$, $-CH_2OR_{10}$, $-CH_2CH_2OR_{10}$, $-OCH_2CF_3$ or $VR_9$;

Z is CH or N;

V is oxygen or sulfur;

$R_9$ is $CH_3$, $-CH_2CH_3$, $-CH_2CH_2OR_{10}$, $-CH_2CO_2R_{10}$, $-\underset{CH_3}{\underset{|}{CH}}CO_2R_{10}$ or $-CH_2CH_2CO_2R_{10}$;

$R_{10}$ is $CH_3$ or $CH_3CH_2$;

$Y_1$ is H, $OCH_3$ or $CH_3$; and

$X_1$ is H, Cl, $OCH_3$, $OCH_2CH_3$ or $CH_3$,

and their agriculturally suitable salts;

providing that (1) $X_1$ and $Y_1$ are not simultaneously H and (2) when R =

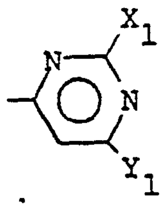

or

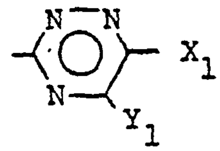

then $R_4$ is H.

2. A compound of Claim 1 wherein $R_4$ is hydrogen.

3. A compound of Claim 2 wherein $R_2$ is hydrogen.

4. A compound of Claim 3 wherein $R_1$ is chlorine.

5. A compound of any of claims 2-4 wherein R is

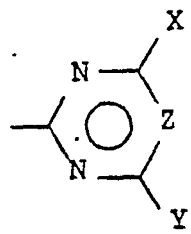

6. A compound of Claim 5 wherein X is $CH_3$ or $-OCH_3$ and Y is $CH_3$, $-OCH_3$ or $-OCH_2CH_3$.

7. N-(4-Methoxy-6-methyl-1,3,5-triazin-2-yl)-N'-(3-pyridinylsulfonyl)carbamimido-thioic acid, methyl ester and its agriculturally suitable salts.

8. N-(3-Pyridinylsulfonyl)-carbonimidodithioic acid, dimethyl ester and its agriculturally suitable salts.

9. A composition for the control of undesired vegetation comprising a herbicidal compound and at least one of (a) a surface active agent and (b) a solid or liquid diluent, characterised in

that said herbicidal compound comprises a compound of general formula I as claimed in any of claims 1-7, or an agriculturally suitable salt thereof.

10. A method for the control of undesirable vegetation by applying to the locus of such vegetation an effective amount of a herbicide characterised in

that said herbicide comprises a compound of general formula I as claimed in any of claims 1-7, or an agriculturally suitable salt thereof.

11. A method of regulating the growth of vegetation by applying to the locus of such vegetation an effective amount of a plant growth regulant, characterised in that said plant growth regulant comprises a compound of general formula I as claimed in any of claims 1-7, or an agriculturally suitable salt thereof.

12. A process for the preparation of a compound of general formula I as defined in claim 1 which comprises reacting a compound of general formula II as defined in claim 1 with an amino- or methylamino-substituted pyrimidine or triazine of general formula

$$H\underset{\substack{|\\R_4}}{N} - R \qquad \text{(III)}$$

wherein R and $R_4$ are as defined in claim 1, in the presence of a strong base.

13. A process for the preparation of a compound of general formula II as defined in claim 1 which comprises reacting together

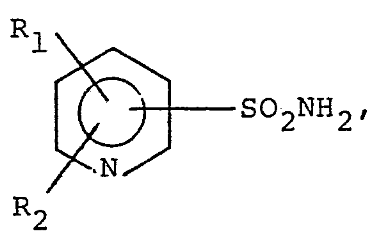, $CS_2$ and $R_3X$

wherein $R_1$, $R_2$ and $R_3$ are as defined in claim 1 and X is halogen, in the presence of an alkali metal hydroxide.